(19) Europäisches Patentamt
European Patent Office
Office européen des brevets

(11) EP 1 674 442 A1

(12) EUROPEAN PATENT APPLICATION
published in accordance with Art. 158(3) EPC

(43) Date of publication:
**28.06.2006 Bulletin 2006/26**

(21) Application number: **04792145.7**

(22) Date of filing: **07.10.2004**

(51) Int Cl.:
***C07C 49/92*** *(1980.01)* ***C07C 69/88*** *(1968.09)*
***C08K 5/00*** *(1990.01)* ***C08L 101/00*** *(1985.01)*
*C07F 5/00* *(1968.09)* *C09K 11/06* *(1974.07)*

(86) International application number:
**PCT/JP2004/014848**

(87) International publication number:
**WO 2005/044770 (19.05.2005 Gazette 2005/20)**

(84) Designated Contracting States:
**DE**

(30) Priority: **07.10.2003 JP 2003348491**
**08.03.2004 JP 2004064178**

(71) Applicant: **Juridical Foundation Osaka Industrial Promotion Organization**
**Osaka-shi,**
**osaka 540-0029 (JP)**

(72) Inventors:
- **MANSEKI, Kazuhiro**
**5620035 (JP)**
- **HASEGAWA, Yasuchika**
**Toyonaka-shi,**
**Osaka 5600055 (JP)**
- **YANAGIDA, Shozo**
**6660133 (JP)**

(74) Representative: **Vossius & Partner**
**Siebertstrasse 4**
**81675 München (DE)**

# (54) HEAT-RESISTANT RARE EARTH COMPLEX

(57) The present invention provides a fluorescent substance excellent in thermal resistance, which can be uniformly dispersed into plastic materials, and has a high fluorescence intensity even after experienced heat history in plastic forming processes. More specifically, the present invention provides a fluorescent substance containing a rare earth complex having benzophenone or benzoyl substituted with an alkyl group, a cycloalkyl group, an acyl group or an alkoxy group having a carbon number of 1 to 20 as a skeletal structure, in which a plurality of rare earth ions are coordinated with one or more types of molecules having a photosensitizing function.

EP 1 674 442 A1

## Description

[Technical Field of the Invention]

The present invention relates to a rare earth complex excellent in thermal resistance, which can be uniformly dispersed into plastic materials, and has a high fluorescence intensity even after experienced heat history in plastic forming processes, a fluorescent substance containing the same, and formed resin materials containing the same.

[Background Art]

Fluorescent substances are used in a variety of applications employing fluorescence emitted from them. They are compounded, for example, in paint or ink depending on purposes.
For example, JP 2002-188026A (Patent Document 1) and JP 2002-201386A (Patent Document 2) disclose fluorescent substances to be compounded in aqueous ink compositions. In these documents, an organic rare earth complex dye consisting of a rare earth element and ligands are described and thenoyltrifluoroacetone, naphthoyltrifluoroacetone, benzoyltrifluoroacetone, methylbenzoyltrifluoroacetone, and the like are listed as ligands.
Recently, it is considered to compound fluorescent substances into plastic materials in order to invest identification information. Plastic materials have been widely used as materials for food trays, industrial resin sheets and the like. For example, fluorescent substances are compounded into plastic materials used for food trays and fluorescence emitted from these trays is detected, whereby a function identical to that of a bar code system is exerted to invest information for a source of food and the like.
In order to detect fluorescence from plastic materials in which fluorescent substances are compounded, it is required to irradiate a light with a specific wavelength, and fluorescence spectra are different depending on the fluorescent substances used. Therefore, information can be invested as a code. Accordingly, a development in code information investing technology is the center of attention.
Since plastic materials are generally formed by softening at high temperatures (e.g., about 300 °C for polycarbonate products), there are required fluorescent substances which do not decompose after heating at higher temperatures in forming and are able to emit sufficient intense fluorescence.
When inorganic fluorescent substances used in cathode ray tubes for color television such as $Y_2O_3$:Eu and the like are used as fluorescent substances to be compounded into plastics, although there is no problem in thermal resistance, it is a problem that inorganic fluorescent substances are impossible to uniformly disperse in plastic materials because they are insoluble in the materials. Further, fluorescence emission can be observed in an organic solvent, but it cannot be observed when compounded in plastic materials.
Therefore, it was considered to use organic rare earth complexes as disclosed in Patent Documents 1 and 2 as fluorescent substances which can be uniformly dispersed in plastic materials. However, since these fluorescent substances are not to intend for compounding into compositions requiring thermal resistance, when they are heated at temperatures needed to form plastic materials, ligands constituting complexes decompose.
Thus, in order to achieve an object to invest plastic products with fluorescence identification information, it is demanded to develop fluorescent substances excellent in thermal resistance, which can be uniformly dispersed into plastic materials, and has a high fluorescence intensity even after experienced heat history in plastic forming processes.

[Disclosure of the Invention]

[Problem to be solved by the Invention]

The object of the present invention is to provide fluorescent substances excellent in thermal resistance, which can be uniformly dispersed into plastic materials, and has a high fluorescence intensity even after experienced heat history in plastic forming processes.

[Means for solving the problem]

The present inventors have made every effort to study with considering the above situation to find that a multinuclear rare earth complex, in which a plurality of rare earth ions are coordinated with additives which are conventionally compounded in plastics, surprisingly exhibit high thermal resistance that cannot be considered in conventional plastics. Consequently, the present invention has been accomplished based on the above findings.
The rare earth complex according to the present invention uses, as a ligand, plastic additives used for ultraviolet absorber. Therefore, its uniform dispersibility in plastic materials is very good, and it dose not decompose in forming plastic materials resulting in sufficient thermal resistance.

In the present invention, a "rare earth ion" means lanthanide ions, and more specifically, 14 lanthanide ions of Ce, Pr, Nd, Pm, Sm, Eu, Gd, Tb, Dy, Ho, Er, Tm, Yb and Lu. Lanthanides usually convert into a trivalent cation, but cerium (Ce) may convert into a tetravalent cation, and europium (Eu) may convert into a divalent cation.

Generally, since rare earth complexes emit fluorescence in a broad visible wavelength range and their fluorescence life times are long, they are very useful. Emission wavelengths for rare earth complexes are, for example, around 645 nm (red) for Sm, around 629 nm (red) for Eu, around 575 nm (yellowish green) for Dy, around 545 nm (green) for Tb. In addition, comparing to fluorescence life time for usual organic fluorescent compounds, of several nanoseconds, it is known that fluorescence life time for rare earth complex, especially complexes of europium (Eu) and terbium (Tb) are about several hundreds microseconds.

JP 11-256148A (Patent Document 3) discloses an illumination material in use for organic EL devises, which comprises phosphorescent substances having a triplet level and a rare earth complex, and an illumination material using a mononuclear rare earth complex in which a single europium (Eu) is coordinated with 3 molecules of dibenzoylmethane and a phosphorescence substance is illustrated. This rare earth complex receives energy from triplet excimers generated in phosphorescent substances by recombination of holes and electrons in an organic emission layer made by vacuum deposition on a substrate, and finally emits fluorescence derived from the rare earth ion.

On the other hand, the rare earth complex according to the present invention is a multinuclear complex having a plurality of rare earth ions and characterized in that it emits sufficient intense fluorescence by irradiating with ultraviolet or visible light (around 300 nm to around 450 nm) even after experienced heat history in plastic forming processes. In addition, since the multinuclear rare earth complex according to the present invention is directly excited by irradiating with ultraviolet or visible light to emit fluorescence, co-substances such as phosphorescent substances having a triplet level are not necessary.

The present invention provides:

(1) a multinuclear rare earth complex characterized in that a plurality of rare earth ions are coordinated with one or more types of molecules having a photosensitizing function to;

(2) the multinuclear rare earth complex described in (1), wherein the molecules having a photosensitizing function further have a vibrational energy quenching-suppressing function;

(3) the multinuclear rear earth complex described in (1), which is represented by the general formula:

$$L_pL'_q(Ln)_rX_s,$$

wherein
L is a ligand having a photosensitizing function represented by the general formula:

[Chemical Formula 1]

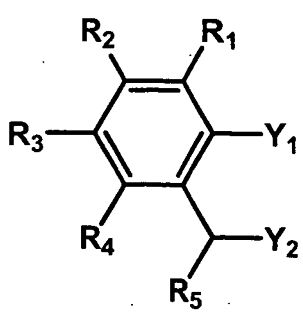

wherein $R_1$, $R_2$, $R_3$, $R_4$ and $R_5$ are independently hydrogen, a hydroxy group, a substituted or unsubstituted amino group,

a substituted or unsubstituted aryl group, a nitro group, a cyano group, an alkyl group or a cycloalkyl group represented by -R, an alkoxy group represented by - OR, or an acyl group represented by -C(C=O)R, where R is a substituted or unsubstituted alkyl group or cycloalkyl group having a carbon number of 1 to 20;
$Y_1$ is -OH; and
$Y_2$ is =O;
p is an integer of 1 to 40;
L' is a ligand which is a hydroxide ion;
q is an integer of 0 to 8;
Ln is a rare earth ion;
r is an integer of 2 to 20, where a plurality of Ln may be the same or different from each other;
X is O, -OH, S, -SH, Se or Te;
s is an integer of 1 to 20, where a plurality of X may be the same or different from each other when s is an integer of 2 to 20; and further, the integers p, r and s have a relationship indicated by the expression:
[Expression 1]

$$1 \leq p/r \leq 4, \quad 1 \leq r/s \leq 4$$

wherein a manner how L is coordinated with Ln:

Coordination Manner (A) where both $Y_1$ and $Y_2$ bind to the identical Ln; Coordination Manner (B) where $Y_1$ and $Y_2$ bind to different Ln, respectively; and a combination thereof, wherein when Ln is coordinated with $Y_1$, a proton leaves from -OH represented by $Y_1$ to form -O-, thereby Ln is coordinated with L via -O-;

(4) the multinuclear rare earth complex described in (3), wherein at least one of substituents $R_1$, $R_2$, $R_3$, $R_4$ and $R_5$ is an alkyl group or a cycloalkyl group represented by -R, an alkoxy group represented by -OR or an acyl group represented by -C(=O)R, where R is a substituted or unsubstituted alkyl group or cycloalkyl group having a carbon number of 1 to 20;

(5) the multinuclear rare earth complex described in (4), wherein $R_5$ is represented by the formula:

[Chemical Formula 2]

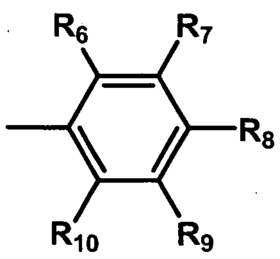

wherein $R_6$, $R_7$, $R_8$, $R_9$ and $R_{10}$ are independently hydrogen, a hydroxy group, a substituted or unsubstituted amino group, a substituted or unsubstituted aryl group, a nitro group, a cyano group, an alkyl group or a cycloalkyl group represented by -R, an alkoxy group represented by -OR, or an acyl group represented by -C(C=O)R, where R is a substituted or unsubstituted alkyl group or cycloalkyl group having a carbon number of 1 to 20, where at least one of $R_1$, $R_2$, $R_3$, $R_4$, $R_6$, $R_7$, $R_8$, $R_9$ and $R_{10}$ is an alkyl group or a cycloalkyl group represented by -R, an alkoxy group represented by -OR, or an acyl group represented by -C(C=O)R, where R is a substituted or unsubstituted alkyl group

or cycloalkyl group having a carbon number of 1 to 20;

(6) the multinuclear rare earth complex described in (4), wherein $R_5$ is an alkyl group or a cycloalkyl group represented by -R, an alkoxy group represented by -OR, or an acyl group represented by -C(C=O)R, where R is a substituted or unsubstituted alkyl group or cycloalkyl group having a carbon number of 1 to 20;

(7) the multinuclear rare earth complex described in (5) or (6), wherein R is a substituted or unsubstituted alkyl group having a carbon number of 6 to 12;

(8) the multinuclear rare earth complex described in (7), wherein R is a substituted or unsubstituted alkyl group having a carbon number of 8 to 12;

(9) the multinuclear rare earth complex described in (1), wherein the rare earth ion is an ion of lanthanide selected from a group consisting of europium (Eu), terbium (Tb), neodymium (Nd), samarium (Sm), erbium (Er) and ytterbium (Yb) or a combination thereof;

(10) the multinuclear rare earth complex described in (5), which is represented by the general formula:

$$L_{10}(Ln)_4X,$$

wherein
L is a ligand represented by the formula:

[Chemical Formula 3]

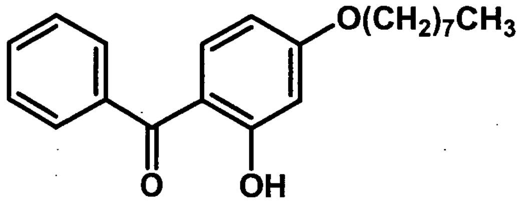

;

Ln is a europium (Eu) ion; and
X is O, and which has the following properties:

Elementary Analysis: as $C_{210}H_{250}O_{31}Eu_4$,

| | | | |
|---|---|---|---|
| Theoretical values | C, 65.04%; | H, 6.50%; | Eu, 15.67% |
| Observed values | C, 64.90%; | H, 6.39%; | Eu, 15.41% |

IR (KBr, $cm^{-1}$): $(\nu_{CH})$2922, $(\nu_{C=C})$1596, $(\nu_{Ph-O})$1243 $^1$H-NMR($CDCl_3$) : δ12.7(1H,s), δ7.6-7.2(3H,m), δ6.5-6.4(5H,d), δ4.0 (2H,t), δ1.8(2H,m), δ0.9(3H,t)
FAB-MS : m/z 3552.1 $[Eu_4(L^-)_9O^{2-}]^+$ ;

(11) the multinuclear rare earth complex described in (5), which is represented by the general formula:

$$L_{10}(Ln)_4X,$$

wherein
L is a ligand represented by the formula:

[Chemical Formula 4]

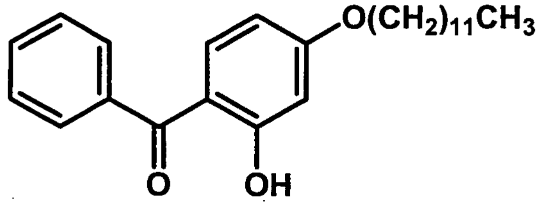

;

Ln is a europium (Eu) ion; and
X is O, and which has the following properties:

Elementary Analysis: as $C_{250}H_{330}O_{31}Eu_4$,

| | | | |
|---|---|---|---|
| Theoretical values | C, 67.64%; | H, 7.49%; | Eu, 13.69% |
| Observed values | C, 67.50%; | H, 7.45%; | Eu, 13.49% |

IR (KBr, $cm^{-1}$): $(\nu_{CH})$2924, $(\nu_{C=C})$1608, $(\nu_{Ph-O})$1247 $^1$H-NMR($CDCl_3$) : δ12.7(1H,s), δ7.6-7.3(3H,m), δ6.5-6.4(5H,d), δ4.0 (2H,t), δ1.8(2H,m), δ0.9(3H,t)
FAB-MS: m/z 4055.9 $[Eu_4(L^-)_9O^{2-}]^+$ ;

(12) the multinuclear rare earth complex described in (6), which is represented by the general formula: $L_{16} L'_8 (Ln)_9X_2$, wherein
L is a ligand represented by the formula:

[Chemical Formula 5]

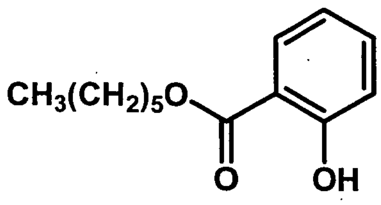

;

L' is $OH^-$;
Ln is a terbium (Tb) ion; and
X is O, and which has the following properties:

Elementary Analysis: as $C_{214}H_{324}O_{72}NTb_9$,

| | | | |
|---|---|---|---|
| Theoretical values | C, 46.79%; | H, 5.93%; | Tb, 26.46% |
| Observed values | C, 46.72%; | H, 5.18%; | Tb, 26.04% |

IR (KBr, $cm^{-1}$): $(\nu_{CH})$2957, 2931, $(\nu_{C=O})$1674, 1637,
$(\nu_{C=C})$1598, $(\nu_{Ph-O})$1243
$^1$H-NMR($CDCl_3$): δ10.9(1H), δ7.9-6.9(4H), δ4.3(2H),
δ1.8(2H), δ1.4(6H), δ0.9(3H)
FAB-MS : m/z 5140.2 $[Tb_9(L^-)_{16}(O^{2-})_2(OH^-)_8+2H^+]^+$ ;

(13) a fluorescent substance containing the multinuclear rare earth complex described in any one of (1) to (12); and

(14) a formed resin material characterized in that the fluorescent substance described in (13) is compounded in plastic polymer.

[Effect of the Invention]

According to the present invention, there are provided a multinuclear rare earth complex excellent in thermal resistance, which can be uniformly dispersed into plastic materials, and has a high fluorescence intensity even after experienced heat history in plastic forming processes, and a fluorescent substance containing the same. Further, according to the present invention, there is also provided formed resin materials characterized in that this fluorescent substance is compounded into plastic polymer.

The rare earth complex according to the present invention can be directly added to plastic materials, paint or ink as a fluorescent substance. A fluorescent substance prepared by mixing an organic dye (for example, coumarin and the like) with the rare earth complex according to the present invention may be added to plastic materials and the like. A formed resin material made by using plastic materials with the fluorescent substance according to the present invention compounded therein may be used as a plastic product invested with illumination or code information. Further, since the fluorescent substance according to the present invention has a high effect on improvement of color rendering, upon compounding this fluorescent substance into a sealing resin used for LED emitting ultraviolet or visible light, a full color LED having a significantly high applicability can be produced.

[Brief Explanation of the Drawings]

[Figure 1] Figure 1 shows a fluorescence spectrum for the Eu complex obtained in Example 1.
[Figure 2] Figure 2 shows an excitation spectrum for the Eu complex prepared in Example 1.
[Figure 3] Figure 3 shows a fluorescence spectrum for the Eu complex prepared in Example 2.
[Figure 4] Figure 4 shows an excitation spectrum for the Eu complex prepared in Example 5.
[Figure 5] Figure 5 shows a fluorescence spectrum for the Tb complex prepared in Example 3.
[Figure 6] Figure 6 shows an excitation spectrum for the Tb complex prepared in Example 3.
[Figure 7] Figure 7 shows an emission spectrum for the formed resin material containing the Eu complex prepared in Example 1.
[Figure 8] Figure 8 shows a graph which illustrates results of DSC measurements for the Eu complex prepared in Example 1 and its ligand itself.

[Best Mode for carrying out the Invention]

The rare earth complex according to the present invention is a multinuclear rare earth complex characterized in that a plurality of rare earth ions are coordinated with one or more types of molecules having a photosensitizing function. The rare earth ion used in the present invention is not particularly limited as far as it is a lanthanide ion, and includes, for example, europium ion $Eu^{3+}$, terbium ion $Tb^{3+}$, cerium ion $Ce^{3+}$, neodymium ion $Nb^{3+}$, samarium ion $Sm^{3+}$, erbium ion $Er^{3+}$, ytterbium ion $Yb^{3+}$ and the like. A plurality of rare earth ions contained in the multinuclear rare earth complex may be the same or different from each other.

Molecules with which the rare earth ions are coordinated are those having a photosensitizing function to sensitize emission from the rare earth ions. In the present invention, a "photosensitizing function" means a function to efficiently transfer energy from irradiation to the rare earth ions.
Such molecules are, for example, a compound having benzophenone or benzoyl as a skeletal structure in which a triplet $\pi$-$\pi^*$ state exists. One or two or more types of such molecules may be contained in the complex.

The rare earth complex according to the present invention has further a vibrational energy quenching-suppressing function. In the present invention, a "vibrational energy quenching-suppressing function" means a function to suppress conversion of emission energy to thermal energy via energy transfer of the excitation state of the fluorescent substance into a vibrational structure of its surrounding media (molecules, solvents, plastics).
Examples include substituents having a long chain alkyl skeletal structure such as an alkyl group, a cycloalkyl group, an acyl group and an alkoxy group, all of which have a carbon number of 6 or greater.

Such a rare earth complex is represented by the general formula: $L_pL'_q(Ln)_rX_s$. Wherein L is a ligand having a photosensitizing function, which is represented by the formula:

[Chemical Formula 6]

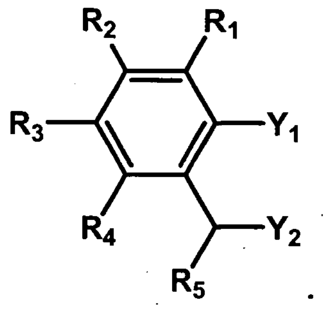

In the above formula, $R_1$, $R_2$, $R_3$, $R_4$ and $R_5$ are independently hydrogen, a hydroxy group, a substituted or unsubstituted amino group, a substituted or unsubstituted aryl group, a nitro group, a cyano group, an alkyl group or a cycloalkyl group represented by -R, an alkoxy group represented by -OR, or an acyl group represented by - C(C=O)R, where R is a substituted or unsubstituted alkyl group or cycloalkyl group having a carbon number of 1 to 20.

In this specification, amino groups are natural amino acids or artificial amino acids, and include, for example, glycine, alanine, leucine, tyrosine and tryptophan.

In this specification, aryl groups include, for example, a phenyl group, a tolyl group, a xylyl group, a biphenyl group, a naphthyl group, an anthryl group and a phenanthryl group.

In this specification, an alkyl group or a cycloalkyl group represented by R include, for example, a methyl group, an ethyl group, a propyl group, an isopropyl group, a hexyl group, an octyl group, a decyl group, a dodecyl group, a cyclopropyl group and a cycloheptyl group.

When the amino group is substituted, the substituents include, for example, an alkyl group, a halogen group, a nitro group, a cyano group and an aryl group.

When the aryl group is substituted, the substituents include, for example, an alkyl group, a halogen group, a nitro group, a cyano group, an alkoxy group and an acyl group.

When the alkyl group is substituted, the substituents include, for example, a halogen group, a nitro group, a cyano group, an amino group, a carboxyl group and an aryl group.

When the cycloalkyl group is substituted, the substituents include, for example, a halogen group, a nitro group, a cyano group, an amino group and an aryl group.

When the alkoxy group is substituted, the substituents include, for example, an alkyl group, a halogen group, an nitro group, a cyano group and an aryl group.

When the acyl group is substituted, the substituents include, for example, an alkyl group, a halogen group, a nitro group, a cyano group, an amino group, an alkoxy group and an aryl group,

Additionally, the alkyl group, the cycloalkyl group, the alkoxy group, the acyl group and the amino group as the above substituents are the same as defined above.

$Y_1$ is -OH; and $Y_2$ is =O.

p is an integer of 1 to 40.

L' is a ligand different from L; and is an ordinary ligand with which a rare earth ion can be coordinated. The ligand L' includes, for example a hydroxide ion.

q is an integer of 0 to 8, and where a plurality of L' may be the same or different from each other when q is an integer of 2 to 8.

Ln is a rare earth ion and is not particularly limited, and includes particularly ions of lanthanides selected from a group consisting of Ce, Pr, Nd, Pm, Sm, Eu, Gd, Tb, Dy, Ho, Er, Tm, Yb and Lu, and these usually exist in a +3 valence state in a complex.

Wherein r is an integer of 2 to 20, and a plurality of Ln may be the same or different from each other.

X is an atom or an atomic group which binds to a plurality of rare earth ions to link them or binds to a single rare earth ion, and a plurality of X do not bind to each other.

X is O, -OH, S, -SH, Se or Te, s is an integer of 1 to 20, and a plurality of X may be the same or different from each other when s is an integer of 2 to 20.

Further, the integers p, r and s have a relationship indicated by the expression:

[Expression 2]

$$1 \leq p/r \leq 4, \quad 1 \leq r/s \leq 4.$$

A manner how Ln is coordinated with L to: Coordination Manner (A) where both $Y_1$ and $Y_2$ bind to the identical Ln; Coordination Manner (B) where $Y_1$ and $Y_2$ bind to different Ln, respectively and a combination thereof, wherein when Ln is coordinated with $Y_1$, a proton leaves from -OH represented by $Y_1$ to form -O-, thereby L coordinates to Ln via -O-.
The coordination manner between L and Ln is explained below referring to a binuclear complex in which a compound of two Ln linked via X are coordinated with one molecule of L.
When Ln is coordinated with L, a proton leaves from -OH represented by $Y_1$ to form -O-, thereby Ln is coordinated with L via -O-. There are Coordination Manner (A) as indicated by the structure (I):
[Chemical Formula 7]

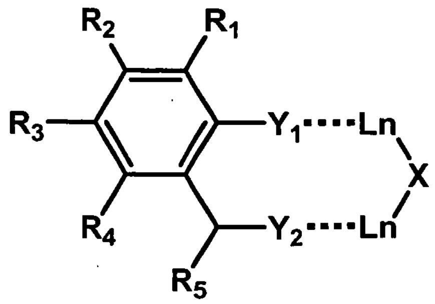

where both $Y_1$ and $Y_2$ bind to the identical Ln; Coordination Manner (B) as indicated by the structure (B):
[Chemical Formula 8]

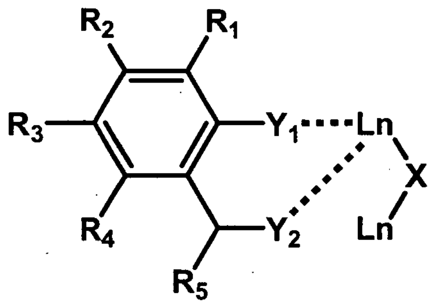

where $Y_1$ and $Y_2$ bind to different Ln each other; and a combination of Coordination Manners (A) and (B).
In addition, a plurality of $Y_1$ and/or $Y_2$ in different ligands L may bind to the identical Ln.
In the complex, the coordination binding site consisting of $Y_1$, $Y_2$ and Ln is in a resonant state.
In the ligand L in the rare earth complex according to the present invention, at least one of $R_1$, $R_2$, $R_3$, $R_4$, $R_6$, $R_7$, $R_8$, $R_9$ and $R_{10}$ is an alkyl group or a cycloalkyl group represented by -R, an alkoxy group represented by -OR, or an acyl group represented by - C(C=O)R, where R is a substituted or unsubstituted alkyl group or cycloalkyl group having a carbon number of 1 to 20.
In particular, R is preferably a substituted or unsubstituted long chain alkyl group having a carbon number of 6 to 20, more preferably a substituted or unsubstituted long chain alkyl group having a carbon number of 8 to 20.

In the first preferred embodiment of the rare earth complex according to the present invention, $R_5$ is a phenyl group represented by the formula:
[Chemical Formula 9]

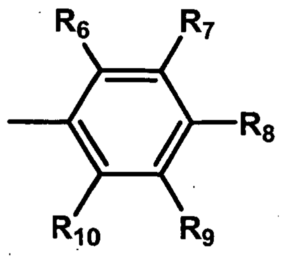

In the formula, $R_6$, $R_7$, $R_8$, $R_9$ and $R_{10}$ are independently hydrogen, a hydroxy group, a substituted or unsubstituted amino group, a substituted or unsubstituted aryl group, a nitro group, a cyano group, an alkyl group or a cycloalkyl group represented by -R, an alkoxy group represented by -OR, or an acyl group represented by - C(C=O)R, where R is a substituted or unsubstituted alkyl group or cycloalkyl group having a carbon number of 1 to 20.

Further, in the first preferred embodiment of the rare earth complex, at least one of $R_1$, $R_2$, $R_3$, $R_4$, $R_6$, $R_7$, $R_8$, $R_9$ and $R_{10}$ is an alkyl group or a cycloalkyl group represented by -R, an alkoxy group represented by -OR, or an acyl group represented by -C(C=O)R, where R is a substituted or unsubstituted alkyl group or cycloalkyl group having a carbon number of 1 to 20.

In this specification, an amino group is a natural amino acid or an artificial amino acid, and includes, for example, glycine, alanine, leucine, tyrosine and tryptophan.
In this specification, an aryl group includes, for example, a phenyl group, a tolyl group, a xylyl group, a biphenyl group, a naphthyl group, an anthryl group and a phenanthryl group.
In this specification, an alkyl group or a cycloalkyl group represented by R includes, for example, a methyl group, an ethyl group, a propyl group, an isopropyl group, a hexyl group, an octyl group, a decyl group, a dodecyl group, a cyclopropyl group and a cycloheptyl group.

When the amino group is substituted, the substituent includes, for example, an alkyl group, a halogen group, a nitro group, a cyano group and an aryl group.
When the aryl group is substituted, the substituent includes, for example, an alkyl group, a halogen group, a nitro group, a cyano group, an alkoxy group and an acyl group.
When the alkyl group is substituted, the substituent includes, for example, a halogen group, a nitro group, a cyano group, an amino group, a carboxyl group and an aryl group.
When the cycloalkyl group is substituted, the substituent includes, for example, a halogen group, a nitro group, a cyano group, an amino group and an aryl group.
When the alkoxy group is substituted, the substituent includes, for example, an alkyl group, a halogen group, an nitro group, a cyano group and an aryl group.
When the acyl group is substituted, the substituent includes, for example, an alkyl group, a halogen group, a nitro group, a cyano group, an amino group, an alkoxy group and an aryl group.
Additionally, the alkyl group, the cycloalkyl group, the alkoxy group, the acyl group and the amino group as the above substituents are the same as defined above.

In the first preferred embodiment of the rare earth complex, L is a ligand represented by the formula:
[Chemical Formula 10]

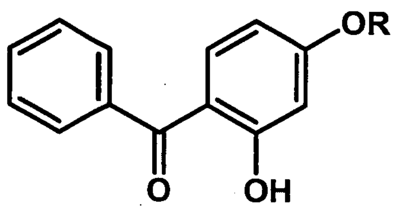

More specifically, L includes, for example, 2-hydroxy-4-octyloxybenzophenone:
[Chemical Formula 11]

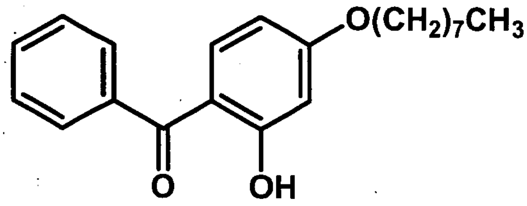

wherein R is octyl, and 4-dodecyloxy-2-hydroxybenzophenone:
[Chemical Formula 12]

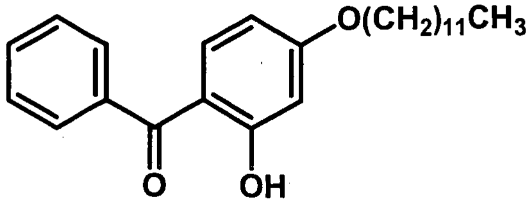

wherein R is dodecyl.
In the second preferred rare earth complex according to the present invention, L includes, for example, hexyl salicylate:
[Chemical Formula 13]

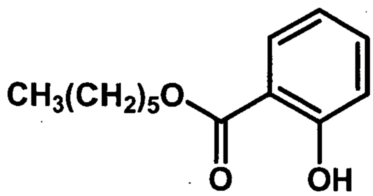

wherein $R_5$ is hexyloxy.
As explained above, in the preferred embodiments, the ligand has benzophenone or benzoyl as a skeletal structure.
These ligands comprise a long chain alkyl group, and due to the existence of this alkyl group, the vicinity of the rare earth complex becomes hydrophobic. When the rare earth complex is coordinated with a water molecule (a polar molecule), this quenches the excitation energy of the ligand having a photosensitizing function to reduce the emission efficiency. For example, in the case of the Eu complex coordinated with the ligands according to the first preferred embodiment, the existence of the long chain alkyl group prevents the rare earth complex from being coordinated with water molecules and, thus, these ligands are considered to have a vibrational energy quenching-suppressing function.
In addition, as will be explained below, in the Eu complex coordinated with this ligand, the excitation spectrum at 615 nm at which emission occurs corresponds to the absorption spectrum of the complex well. Accordingly, in the

present complex, the ligand absorbs the photo energy and energy transfer occurs from the ligand to the rare earth ion to emit a light and, thus, the present ligand is considered to have a photosensitizing function.

The present complex may be prepared, for example, by mixing a compound which will be a ligand and a rare earth compound such as rare earth metal nitrate or rare earth metal acetate in the presence of, for example, triethylamine or lithium hydroxide.

The present complex thus prepared is insoluble in water, but very soluble in nonpolar solvent such as hexane and chloroform due to the existence of a long chain alkyl in a part of the structure. Further, it is slightly soluble in polar solvent such as methanol and acetone. Thereby, it is expected that it may be added to and well dispersed in polymer as a raw material of plastics.

The present rare earth complex may be solely added to plastic materials as a fluorescent substance. In addition, a mixture of the present rare earth complex and an organic dye for changing color (e.g., coumarin) may also be used as a fluorescent substance.

Further, the present invention also provides a formed resin material characterized in that the rare earth complex according to the present invention is compounded into plastic polymers.

The plastic polymer in which the rare earth complex is to be compounded is not limited, but includes, for example, a polyethylene resin, a polypropylene resin, a poly(vinyl chloride) resin, a urea resin, a fluorine resin, a polyester resin, a polyamide resin, a polyacetal resin, a polycarbonate resin, a polyallylate resin, a polysulfone resin, a polyphenylenesulfide resin, a polyethersulfone resin, a polyallylsulfone resin, a polytetrafluoroethylene resin, a phenol resin, an unsaturated polyester resin, an epoxy resin, a polyimide resin and a polyamideimide resin.

A method for forming is not particularly limited, but includes, for example, injection molding, blow molding, compression molding, extrusion forming, reaction forming, blow forming, heat forming, FRP forming and the like. In these methods, forming is usually carried out at 200 °C or higher and, at a higher temperature of about 300 °C for polycarbonate products.

The present complex has an excellent thermal resistance. More specifically, the Eu complex represented by the above formula exhibits thermal stability up to about 310 °C as measured by DSC (that is, the decomposition temperature is about 310 °C).

In addition, the Tb complex described above exhibits thermal stability at least up to about 200 °C. Further, the Eu complex and the Tb complex are durable against water and acids, and excellent in climatic resistance due to a long alkyl chain in the ligand.

The rare earth complex according to the present invention maintains strong emission intensity after forming even when it has experienced heat history in the forming process.

[Examples]

The present invention will be further specifically explained referring to the following Examples, but it should not be understood that these Examples limit the scope of the present invention.

A. Synthesis of Rare Earth Complex

Example 1: Synthesis of Eu tetranuclear complex (I)

Into methanol (100 mL), added were the ligand 2-hydroxy-4-octyloxybenzophenone (a polymer additive, seesorb 102; SHIPRO KASEI KAISHA Ltd.) represented by the formula:

[Chemical Formula 14]

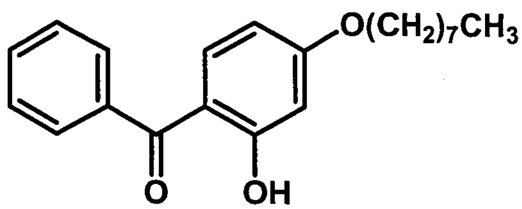

(0.6 g, 1.84 mmol) and a methanol solution of triethylamine (3.48 mL, 1.84 mmol), and after stirring for several minutes, a methanol solution (10 mL) of $Eu(NO_3)_3$·hexahydrate (0.32 g, 0.735 mmol) was added, and the mixture was stirred at room temperature for 2 hours. Yellow powdery crystals were obtained by suction filtration.

After washing the obtained yellow powdery crystals with methanol several times, they were analyzed by elementary analysis, IR, $^1$H-NMR and FAB-MS. Results for these analyses are shown below.

$[Eu_4(L^-)_{10}O^{2-}]$ (Presumed formulation)

| | | | |
|---|---|---|---|
| Elementary Analysis: as $C_{210}H_{250}O_{31}Eu_4$, | | | |
| Theoretical values | C, 65.04%; | H, 6.50%; | Eu, 15.67% |
| Observed values | C, 64.90%; | H, 6.39%; | Eu, 15.41% |

IR (KBr, $cm^{-1}$): ($\nu_{CH}$)2922, ($\nu_{C=C}$)1596, ($\nu_{Ph=O}$)1243 $^1$H-NMR($CDCl_3$) : δ12.7(1H,s), δ7.6-7.2(3H,m), δ6.5-6.4(5H,d), δ4.0 (2H,t), δ1.8(2H,m), δ0.9(3H,t)

FAB-MS : m/z 3552.1 $[Eu_4(L^-)_9O^{2-}]^+$.

A Gd complex and a Tb complex were synthesized according to the above procedure but $Gd(NO_3)_3$·hexahydrate or $Tb(NO_3)_3$·hexahydrate was used in place of $Eu(NO_3)_3$·hexahydrate, and FAB-MS measurement was carried out. Molecular weights for each of rare earth elements and characteristic fragment peaks for each of the rare earth complex are shown in Table 1.

[Table 1]

| Rare Earth Elements | Molecular Weight | Fragment Peak |
|---|---|---|
| Eu | 152.0 | 3552.1 |
| Gd | 157.3 | 3573.3 |
| Tb | 158.9 | 3580.0 |

Since properties of rare earth elements (ionic radius, coordination manner, etc.) are generally very similar to each other, it is supposed that the complexes formed have the same style. That is, it is supposed that only the center metals are replaced in those complexes.

For example, when comparing the Eu complex with the Gd complex, the differences in the fragment peaks and the molecular weights therebetween are about 21 and 5.3, respectively. Under the assumption where only the center metals are replaced in the complexes, a number of the center metals in one complex was calculated to be 4 from 21.2/5.3. Similarly, the number of the center metals was calculated to be 4 from comparisons between the Eu complex and the Tb complex, and the Gd complex and the Tb complex.

Yan et al. [C.-H Yan et al., Inorg. Chem. 41 (2002), 6802] (Non-Patent Document 1) indicated that a rare earth complex synthesized according to a process similar to that of Example 1 has a tetranuclear cross-linking structure comprising oxo linkages, which is represented by the general formula: $Ln_4O$, wherein Ln is a rare earth ion, based on X-ray structural analysis. From this knowledge and the above FAB-MS measurement results, the above Eu complex was suggested to be a Eu tetranuclear complex having a $Eu_4O$ cross-linking structure.

In addition, from the results of IR measurement, there is no peak around 3400 $cm^{-1}$ derived from water molecules and, thereby, it is not considered that water molecules are contained in the complex as crystal water or ligands. This is consistent with the results of DSC described bellow.

Based on the above analysis results, when a ligand represented by:

[Chemical Formula 15]

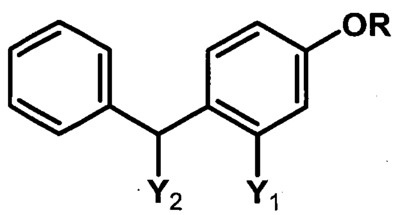

wherein R is an alkyl group having a carbon number of 8 to 12, and both $Y_1$ and $Y_2$ are O, is indicated by the following

illustration:

[Chemical Formula 16]

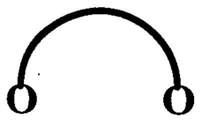

,

the Eu tetranuclear complex according to the present invention is presumed to be represented, for example, by

[Chemical Formula 17]

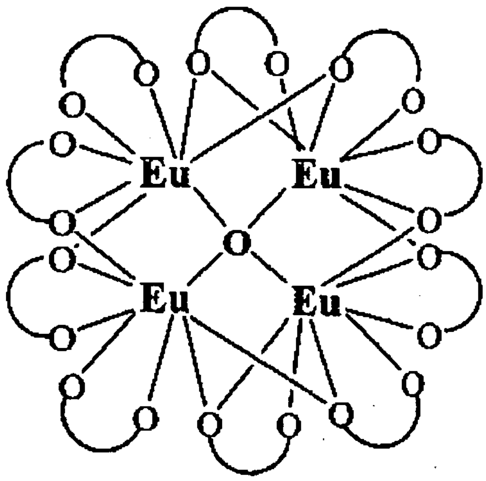

The above complex structure is merely one example to assist understanding of the structure of the present complex and, therefore, the present complex is not limited to those having this structure.

Example 2: Synthesis of Eu tetranuclear complex (II)

Into methanol (100 mL), added were the ligand 4-dodecyloxy-2-hydoxybenzophenone (a polymer additive, seesorb 102; SHIPRO KASEI KAISHA Ltd.) represented by the formula:

[Chemical Formula 18]

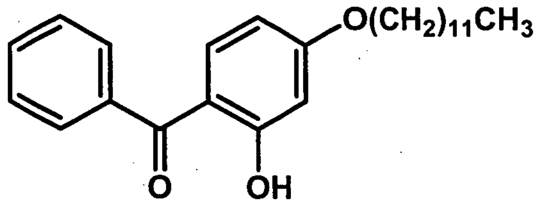

(0.6 g, 1.57 mmol) and a methanol solution of triethylamine (2.97 mL, 1.57 mmol), and after stirring for several minutes, a methanol solution (10 mL) of $Eu(NO_3)_3$·hexahydrate (0.280 g, 0.627 mmol) was added, and the mixture was stirred at room temperature for 2 hours. Yellow powdery crystals were obtained by suction filtration.

After washing the obtained yellow powdery crystals with methanol several times, they were analyzed by elementary

analysis, IR, $^1H$-NMR and FAB-MS. Results for these analyses are shown below.

$[Eu_4(L^-)_{10}O^{2-}]$ (Presumed formulation)

| | | | |
|---|---|---|---|
| Elementary Analysis: as $C_{250}H_{330}O_{31}Eu_4$, | | | |
| Theoretical values | C, 67.64%; | H, 7.49%; | Eu, 13.69% |
| Observed values | C, 67.50%; | H, 7.45%; | Eu, 13.49% |

IR (KBr, $cm^{-1}$): $(\nu_{CH})$2924, $(\nu_{C=C})$1608, $(\nu_{Ph-O})$1247 $^1H$-NMR($CDCl_3$) : δ12.7(1H,s), δ7.6-7.3(3H,m), δ6.5-6.4(5H,d), δ4.0 (2H,t), δ1.8(2H,m), δ0.9(3H,t)

FAB-MS: m/z 4055. 9 $[Eu_4(L^-)_9O^{2-}]^+$.

Similar to the Eu tetranuclear complex (I) synthesized in Example 1, this complex was suggested to be a Eu tetranuclear complex having a $Eu_4O$ cross-linking structure.

Example 3: Synthesis of Tb nonanuclear complex (III)

To a methanol solution of a ligand hexyl salicylate represented by the general formula:

[Chemical Formula 19]

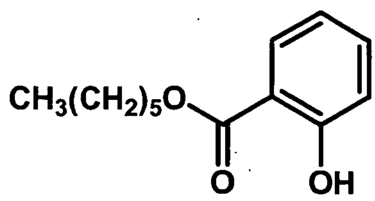

(0.600 g, 2.70 mmol), an equivalent mole amount of triethylamine (0.270 g, 2.70 mmol) was added, and after stirring for a while, a methanol solution (10 mL) of $Tb(NO_3)_3$·hexahydrate (0.600 g, 1.35 mmol) was added, and stirred at room temperature for 30 minutes. White crystals were obtained by suction filtration.

After washing the obtained yellow powdery crystals with methanol several times, they were analyzed by elementary analysis, IR, $^1H$-NMR and FAB-MS. Results for these analyses are shown below.

$[Tb_9(L^-)_{16}(O^{2-})_2(OH^-)_8]^-[(C_2H_5)_3NH]^+\cdot 14H_2O$ (presumed formulation)

| | | | |
|---|---|---|---|
| Elementary Analysis: as $C_{214}H_{324}O_{72}NTb_9$, | | | |
| Theoretical values | C, 46.79%; | H, 5.93%; | Tb, 26.46% |
| Observed values | C, 46.72%; | H, 5.18%; | Tb, 26.04% |

IR (KBr, $cm^{-1}$): $(\nu_{CH})$2957, 2931, $(\nu_{C=O})$1674, 1637,
$(\nu_{C=C})$1598, $(\nu_{Ph-O})$1243
$^1H$-NMR($CDCl_3$): δ10.9(1H), δ7.9-6.9(4H), δ4.3(2H),
δ1.8(2H), δ1.4(6H), δ0.9(3H)
FAB-MS: m/z 5140.2 $[Tb_9(L^-)_{16}(O^{2-})_2(OH^-)_8+2H^+]^+$

A Sm complex, a Eu complex, Gd complex and a Yb complex were synthesized according to the above procedure but $Sm(NO_3)_3$·hexahydrate, $Eu(NO_3)_3$·hexahydrate, $Gd(NO_3)_3$·hexahydrate or $Yb(NO_3)_3$·hexahydrate was used in place of $Tb(NO_3)_3$·hexahydrate, and FAB-MS measurement was carried out. Molecular weights for each of rare earth elements and characteristic fragment peaks for each of the rare earth complex are shown in Table 2.

[Table 2]

| Rare Earth Elements | Molecular Weight | Fragment Peak |
|---|---|---|
| Sm | 150.4 | 5063.9 |
| Eu | 152.0 | 5077.7 |
| Gd | 157.3 | 5125.8 |
| Tb | 158.9 | 5140.2 |
| Yb | 173.0 | 5267.9 |

Similar to Example 1, when comparing the Tb complex with other complexes, the difference in the fragment peaks corresponds to 9 rare earth elements and, thus, the number of the center metals was calculated to be 9.

From results of the above FAB-MS measurements, this complex was suggested to be a Tb nonanuclear complex having a polynuclear structure. With considering that Non-Patent Document 1 indicates the $Ln_4O$ oxo cross-linking structure, it is supposed that this complex has a sandwich structure where one Tb molecule resides between two of $Tb_4O$ cross-linking structures.

In addition, in this complex, it was presumed that quarternized triethylamime ionically bound as counter cations to form a salt.

Further, not indicated above, from results of IR measurement, a broad peak was observed around 3400 $cm^{-1}$ derived from $H_2O$. Therefore, it is suggested that this complex contains water molecules. From results of DSC measurement described in Example 10, it was demonstrated that these water molecules were crystal water adsorbing to the complex in a salt form.

B. Fluorescence Properties of Rare Earth Complex in Organic Solvent

Example 4: Fluorescence properties of the Eu tetranuclear complex (I) in hexane

A fluorescence spectrum of the Eu complex (I) prepared in Example 1 was measured in hexane. Figure 1 shows the fluorescence spectrum and Figure 2 shows the excitation spectrum.

Measurements were carried out at a concentration of 1 x $10^{-4}$ M, slit widths of 5 nm:5 nm, and the excitation wavelength was 385 nm for the fluorescence spectrum and the monitoring wavelength was 614 nm for the excitation spectrum.

From Figure 1 and Figure 2, it is confirmed that the present complex exhibits fluorescence in hexane. In the fluorescence spectrum, the peak at 614 nm is derived from $^5Do \rightarrow ^7F_2$ transition of Eu(III).

From the result of the excitation spectrum, when comparing with its absorption spectrum, it is considered that this peak is derived from the ligands and, thus, this complex emits light through photosensitization. In addition, it is found that the peak shown around 385 nm is derived from a $\pi$-$\pi$* transition of the ligands, which is observed as a result of complex formation, and the complex emits light better when excited at this wavelength.

Example 5: Fluorescence properties of Eu tetranuclear complex (II) in hexane

Next, a fluorescence spectrum of the Eu complex (II) prepared in Example 2 was measured in hexane. Figure 3 shows the fluorescence spectrum and Figure 4 shows the excitation spectrum.

Measurements were carried out at a concentration of 1 x $10^{-4}$ M, slit widths 5 nm:5 nm, and the excitation wavelength was 385 nm for the fluorescence spectrum and the monitoring wavelength was 614 nm for the excitation spectrum.

Example 6: Fluorescence Properties of the Tb nonanuclear complex (III) in methanol

Next, a fluorescence spectrum of the Tb complex (III) prepared in Example 3 was measured in hexane. Figure 5 shows the fluorescence spectrum and Figure 6 shows the excitation spectrum.

Measurements were carried out at a concentration of 1 x $10^{-4}$ M, slit widths 2.5 nm:2.5 nm, and the excitation wavelength was 360 nm for the fluorescence spectrum and the monitoring wavelength was 545 nm for the excitation spectrum.

C. Fluorescence Properties of Rare Earth Complex in Formed Resin Material

Example 7: Fluorescence properties of the Eu tetranuclear complex (I) in a formed polypropylene materials

The Eu complex (I) prepared in Example 1 was compounded in polypropylene at a concentration of 100 ppm, and it was injection-molded at a resin temperature of about 200 °C to form a plate (size about 3 mm x about 48 mm x about 83 mm). Then, the emission spectrum of this plate was measured by using a PGP detector No.4 Type B (Refraction type) with an excitation wavelength at 385 nm. Results are shown in Figure 7. As shown in this figure, it is found that the formed resin material in which the present rare earth complex is compounded exhibits good fluorescence emission even after experienced a heat history at a high temperature.

D. Thermal Stability of Rare Earth Complex

Example 8: Thermal Stability of the Eu tetranuclear complex (I) in the air
In order to determine thermal stability of the Eu complex (I) prepared in Example 1, DSC measurement was carried out from room temperature to 500 °C at a heating rate of 10 °C/minute using an aluminum pan. Results are shown in Figure 8. In addition, measurement data for ligand itself are also shown for comparison.

It is found that for the ligand itself, a peak derived from its melting point is observed around 50 °C, while in the complex, such peak is not detected.
Based on the measurement result (the temperature at which a peak rises), the decomposition temperature of this complex (I) was found to be 310 °C.
Since regardless of the ligand itself melting around 50 °C, the complex did not decompose up to over 300 °C, it is considered that complexation improves thermal stability.
It is revealed that the present complexes have much higher thermal stability than a trifluoroacethylacetone Eu complex, $Eu(hfac)_3$, which is known to have a decomposition temperature in the air of 220 °C.

The complex (I) was heated at 250 °C for about 10 minutes in the air, and after cooling, it was irradiated with a UV lamp (365 nm), and emission was observed visually.

Example 9: Thermal stability of the Eu tetranuclear complex (II) in the air
Similar to Example 1, DSC measurement was carried out for the complex (II) prepared in Example 2. The decomposition temperature of this complex was found to be 320 °C.

Example 10: Thermal stability of the Tb nonanuclear complex (III) in the air
Similar to Example 1, DSC measurement was carried out for the complex (III) prepared in Example 2. The decomposition temperature of this complex was found to be 200 °C.
In addition, an endothermic peak was observed around 90 °C. With combining the result of IR measurement, it is considered that this endothermic peak derives from desorbing of water molecules existing in the complex.
Hasegawa et al. [Y. Hasegawa et al., J. Phys. Chem. 100 (1996) 10201] (Non-Patent Document 2) reported that when water molecules existing in a Nd complex were ligands, the endothermic peak was observed in a higher temperature region around 130 to 160 °C. Since properties of rare earth ions do not greatly vary, it is considered that when water molecules are ligands, the endothermic peak for the Tb complex is also in a temperature region around 150 °C. Therefore, it is considered that the endothermic peak around 90 °C in the Tb nonanuclear complex (III) derives from desorption of crystal water adsorbing to the crystals rather than leaving of water molecules as ligands.

Example 11: Thermal stability in fluorescence properties of the Eu complex in a polymer thin film
Since it is revealed from the above DSC measurements that the Eu tetranuclear complex does not decompose even after heating at 300 °C or higher in the air, effects of the heat history on the fluorescence properties were further investigated.
Specifically, a fluorescent polymer was prepared by uniformly dispersing the Eu complex (I) or the Eu complex (II) into polyphenylsilsesquioxane (PPSQ). The mixing ratio between PPSQ and the Eu complexes was 90 wt%/10 wt%.
This fluorescent polymer was smeared on a glass substrate to form thin films. In a fluorescence life time measurement, since a film thickness does not affect the measurement, the thickness may be in a range where measurement is possible. For each of these thin films, fluorescence life times were measured at 25 °C by exciting at 380 nm and monitored at 615 nm. Further, these films were heated at 150 °C, 200 °C and 250 °C for 5 minutes in a furnace, and after cooling to room temperature, fluorescence life times were measured similarly. Results are shown in Figure 3.
Additionally, since the decomposition temperature of PPSQ itself is 500 °C or higher, this does not affect this experiment.

[Table 3] Fluorescence Life Times for Eu Complexes after Heating PPSQ Thin Films

| Heating Temperature for Thin Film | Fluorescence Life Time (τ) | |
|---|---|---|
| | Eu Complex (I) | Eu Complex (II) |
| 25 °C | 0.38 ms | 0.39 ms |
| 150 °C | 0.40 ms | 0.42 ms |
| 200 °C | 0.38 ms | 0.40 ms |
| 250 °C | 0.37 ms | 0.36 ms |

Based on the above results, it is revealed that the fluorescence life times for the Eu tetranuclear complexes prepared by the present invention do not change after heating at 250 °C. That is, it is confirmed that these are stable as a complex at 250 °C. For comparison, polymer thin films were formed using a conventional complex $Eu(hfac)_3$ and they were heated at 250 °C. A fluorescence life time could not be measured due to blackening.
This difference is consistent with the difference in the decomposition temperature in the air (300 °C or higher for the Eu

complex (I) and the Eu complex (II), 220 °C for $Eu(hfac)_3$).

[Industrial Applicability]

The fluorescent substances according to the present invention may be compounded into materials requiring thermal resistance, such as plastic materials to be formed at high temperatures, to invest plastic products with fluorescence identification information.

## Claims

1. A multinuclear rare earth complex **characterized in that** a plurality of rare earth ions are coordinated with one or more types of molecules having a photosensitizing function.

2. The multinuclear rare earth complex according to claim 1, wherein the molecules having a photosensitizing function further have a vibrational energy quenching-suppressing function.

3. The multinuclear rare earth complex according to claim 1, which is represented by the general formula:

$$L_pL'_q(Ln)_rX_s,$$

wherein
L is a ligand having a photosensitizing function represented by the general formula:

[Chemical Formula 1]

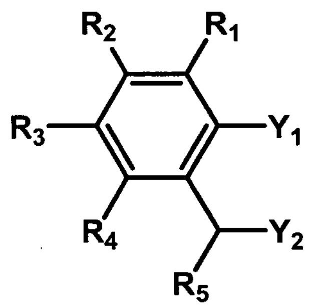

wherein $R_1$, $R_2$, $R_3$, $R_4$ and $R_5$ are independently hydrogen, a hydroxy group, a substituted or unsubstituted amino group, a substituted or unsubstituted aryl group, a nitro group, a cyano group, an alkyl group or a cycloalkyl group represented by -R, an alkoxy group represented by - OR, or an acyl group represented by -C(C=O)R, where R is a substituted or unsubstituted alkyl group or cycloalkyl group having a carbon number of 1 to 20;
$Y_1$ is -OH; and
$Y_2$ is =O;
p is an integer of 1 to 40;
L' is a ligand which is a hydroxide ion;
q is an integer of 0 to 8;
Ln is a rare earth ion;
r is an integer of 2 to 20, and a plurality of Ln may be the same or different from each other;
X is O, -OH, S, -SH, Se or Te;
s is an integer of 1 to 20, and a plurality of X may be the same or different from each other when s is an integer of 2 to 20; and further, the integers p, r and s have a relationship indicated by the expression:

[Expression 1]

$$1 \leq p/r \leq 4,\ 1 \leq r/s \leq 4$$

wherein a manner how Ln is coordinated with L: Coordination Manner (A) where both $Y_1$ and $Y_2$ bind to the identical Ln; Coordination Manner (B) where $Y_1$ and $Y_2$ bind to different Ln, respectively; and a combination thereof, wherein when Ln is coordinated with $Y_1$, a proton leaves from -OH represented by $Y_1$ to form -O-, thereby Ln is coordinated with L via -O-.

**4.** The multinuclear rare earth complex according to claim 3, wherein at least one of substituents R1, $R_2$, $R_3$, $R_4$ and $R_5$ is an alkyl group or a cycloalkyl group represented by -R, an alkoxy group represented by -OR or an acyl group represented by -C(=O)R, where R is a substituted or unsubstituted alkyl group or cycloalkyl group having a carbon number of 1 to 20.

**5.** The multinuclear rare earth complex according to claim 4, wherein $R_5$ is represented by the formula:

[Chemical Formula 2]

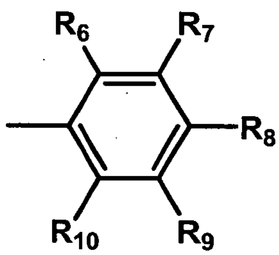

wherein $R_6$, $R_7$, $R_8$, $R_9$ and $R_{10}$ are independently hydrogen, a hydroxy group, a substituted or unsubstituted amino group, a substituted or unsubstituted aryl group, a nitro group, a cyano group, an alkyl group or a cycloalkyl group represented by -R, an alkoxy group represented by -OR, or an acyl group represented by -C(C=O)R, where R is a substituted or unsubstituted alkyl group or cycloalkyl group having a carbon number of 1 to 20, where at least one of $R_1$, $R_2$, $R_3$, $R_4$, $R_6$, $R_7$, $R_8$, $R_9$ and $R_{10}$ is an alkyl group or a cycloalkyl group represented by -R, an alkoxy group represented by -OR, or an acyl group represented by -C(C=O)R, where R is a substituted or unsubstituted alkyl group or cycloalkyl group having a carbon number of 1 to 20.

**6.** The multinuclear rare earth complex according to claim 4, wherein $R_5$ is an alkyl group or a cycloalkyl group represented by -R, an alkoxy group represented by - OR, or an acyl group represented by -C(C=O)R, where R is a substituted or unsubstituted alkyl group or cycloalkyl group having a carbon number of 1 to 20.

**7.** The multinuclear rare earth complex according to claim 5 or 6, wherein R is a substituted or unsubstituted alkyl group having a carbon number of 6 to 12.

**8.** The multinuclear rare earth complex according to claim 7, wherein R is a substituted or unsubstituted alkyl group having a carbon number of 8 to 12.

**9.** The multinuclear rare earth complex according to claim 1, wherein the rare earth ion is an ion of lanthanide selected from a group consisting of europium (Eu), terbium (Tb), neodymium (Nd), samarium (Sm), erbium (Er) and ytterbium (Yb) or a combination thereof.

**10.** The multinuclear rare earth complex according to claim 5, which is represented by the general formula:

$L_{10}(Ln)_4X$,

wherein
L is a ligand represented by the formula:

[Chemical Formula 3]

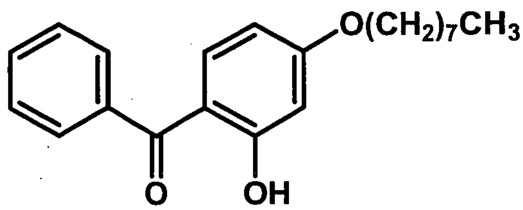

;

Ln is a europium (Eu) ion; and
X is O, and which has the following properties:

| | | | |
|---|---|---|---|
| Elementary Analysis: as $C_{210}H_{250}O_{31}Eu_4$, | | | |
| Theoretical values | C, 65.04%; | H, 6.50%; | Eu, 15.67% |
| Observed values | C, 64.90%; | H, 6.39%; | Eu, 15.41% |

IR (KBr, $cm^{-1}$): ($\nu_{CH}$)2922, ($\nu_{C=C}$)1596, ($\nu_{Ph-O}$)1243 $^1H$-NMR($CDCl_3$) : δ12.7(1H,s), δ7.6-7.21(3H,m), δ6.5-6.4(5H, d), δ4.0(2H,t), δ1.8(2H,m), δ0.9(3H,t)
FAB-MS : m/z 3552.1 $[Eu_4(L^-)_9O^{2-}]^+$.

**11.** The multinuclear rare earth complex according to claim 5, which is represented by the general formula:

$L_{10}(Ln)_4X$,

wherein
L is a ligand represented by the formula:

[Chemical Formula 4]

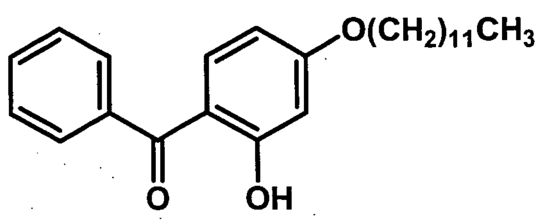

;

Ln is a europium (Eu) ion; and
X is O, and which has the following properties:

| | | | |
|---|---|---|---|
| Elementary Analysis: as $C_{250}H_{330}O_{31}Eu_4$, | | | |
| Theoretical values | C, 67.64%; | H, 7.49%; | Eu, 13.69% |
| Observed values | C, 67.50%; | H, 7.45%; | Eu, 13.49% |

IR (KBr, $cm^{-1}$): ($\nu_{CH}$)2924, ($\nu_{C=C}$)1608, ($\nu_{Ph\text{-}O}$)1247 $^1$H-NMR($CDCl_3$) : δ12.7(1H,s), δ7.6-7.3(3H,m), δ6.5-6.4(5H,d) δ4.0(2H,t), δ1.8(2H,m), δ0.9(3H,t)
FAB-MS: m/z 4055.9 $[Eu_4(L^-)_9O^{2-}]^+$.

**12.** The multinuclear rare earth complex according to claim 6, which is represented by the general formula:

$$L_{16}\,L'_8\,(Ln)_9X_2,$$

wherein
L is a ligand represented by the formula:

[Chemical Formula 5]

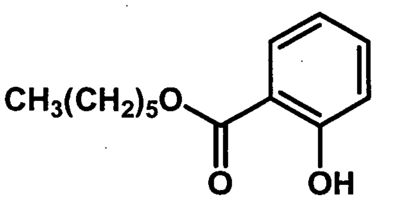
;

L' is $OH^-$;
Ln is a terbium (Tb) ion; and
X is O, and which has the following properties:

| Elementary Analysis: as $C_{214}H_{324}O_{72}NTb_9$, | | | |
|---|---|---|---|
| Theoretical values | C, 46.79%; | H, 5.93%; | Tb, 26.46% |
| Observed values | C, 46.72%; | H, 5.18%; | Tb, 26.04% |

IR (KBr, $cm^{-1}$): ($\nu_{CH}$)2957, 2931, ($\nu_{C=O}$)1674, 1637, ($\nu_{C=C}$)1598, ($\nu_{Ph\text{-}O}$)1243
$^1$H-NMR($CDCl_3$): δ10.9(1H), δ7.9-6.9(4H), δ4.3(2H),
δ1.8(2H), δ1.4(6H), δ0.9 (3H)
FAB-MS: m/z 5140.2 $[Tb_9(L^-)_{16}(O^{2-})_2(OH^-)_8+2H^+]^+$.

**13.** A fluorescent substance containing the multinuclear rare earth complex according to any one of claims 1 to 12.

**14.** A formed resin materials **characterized in that** the fluorescent substance according to claim 13 is compounded in a plastic polymer.

Fig. 1

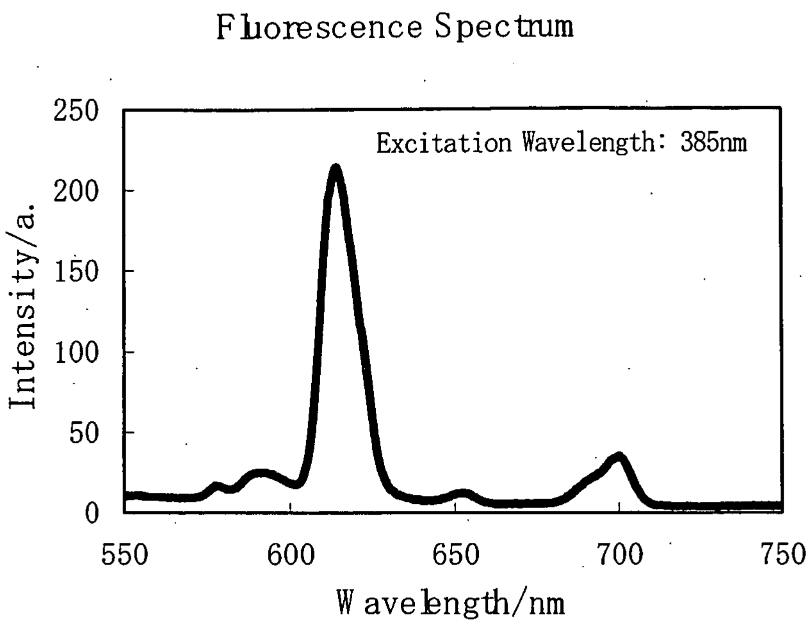
Fluorescence Spectrum
Excitation Wavelength: 385nm
Intensity/a.
250
200
150
100
50
0
550
600
650
700
750
Wavelength/nm

Fig. 2

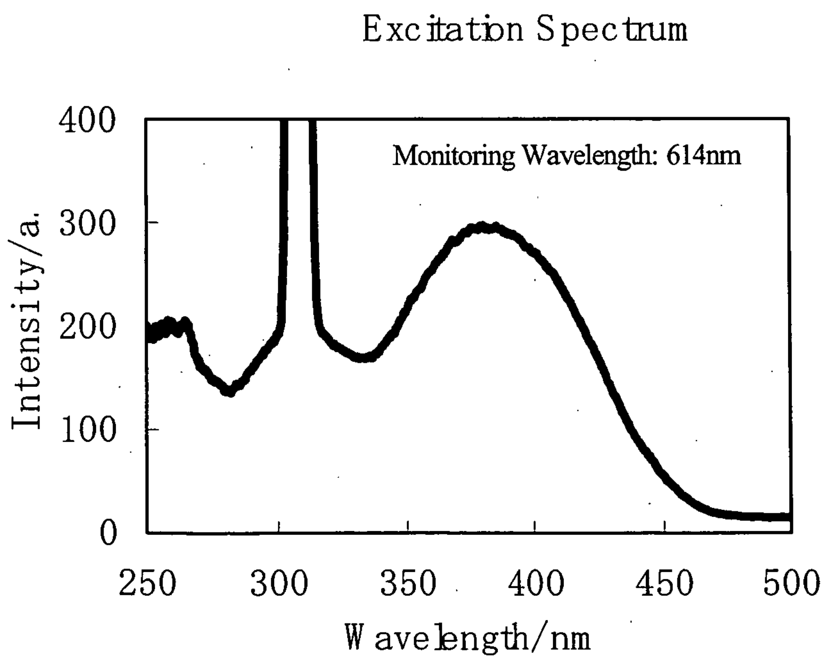
Excitation Spectrum
Monitoring Wavelength: 614nm
Intensity/a.
400
300
200
100
0
250
300
350
400
450
500
Wavelength/nm

Fig. 3

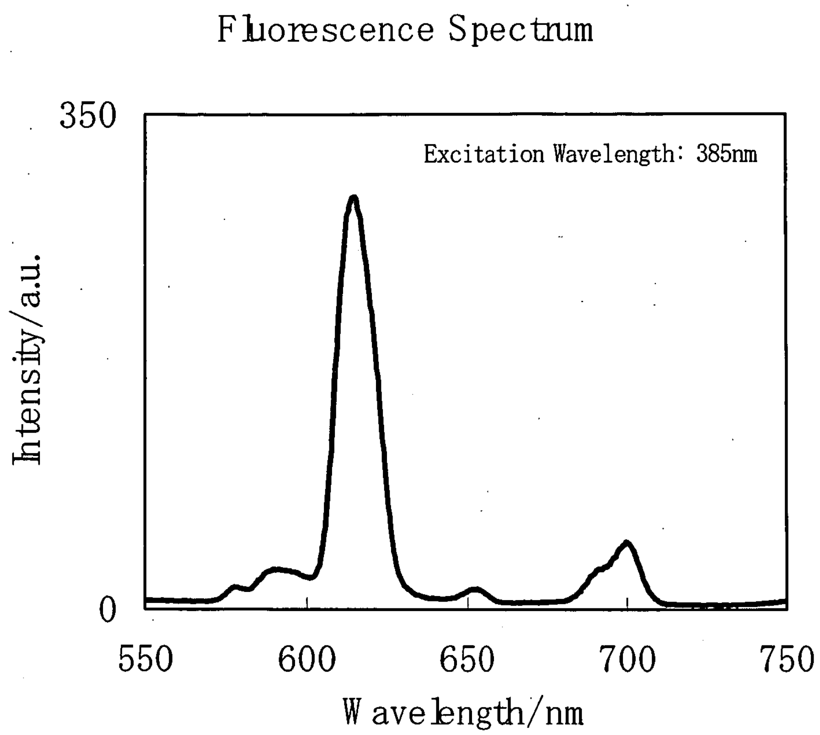
Fluorescence Spectrum
350
0
Excitation Wavelength: 385nm
Intensity/a.u.
550
600
650
700
750
Wavelength/nm

Fig. 4

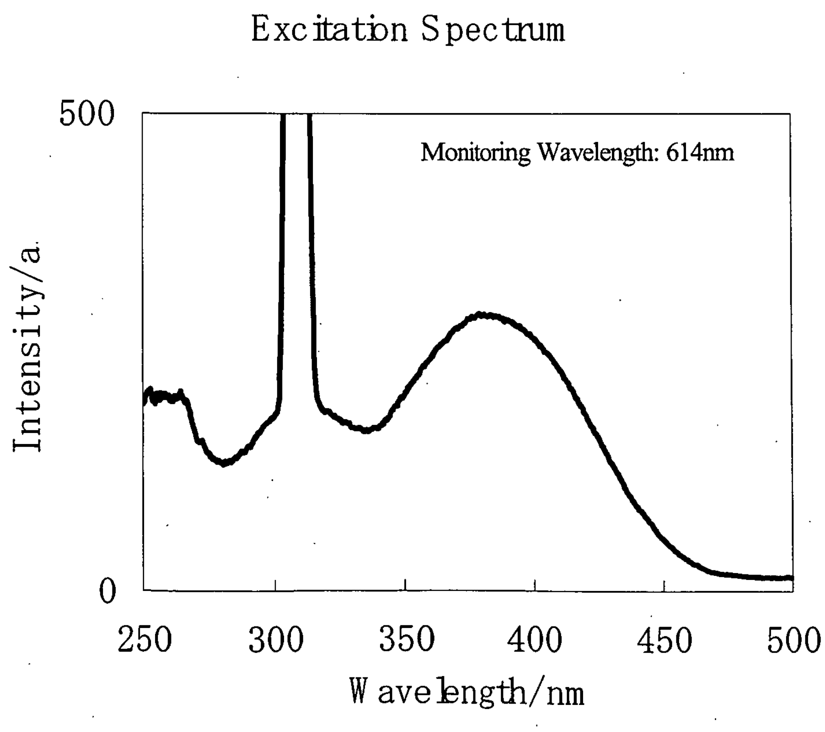
Excitation Spectrum
500
0
Monitoring Wavelength: 614nm
Intensity/a.
250
300
350
400
450
500
Wavelength/nm

Fig. 5

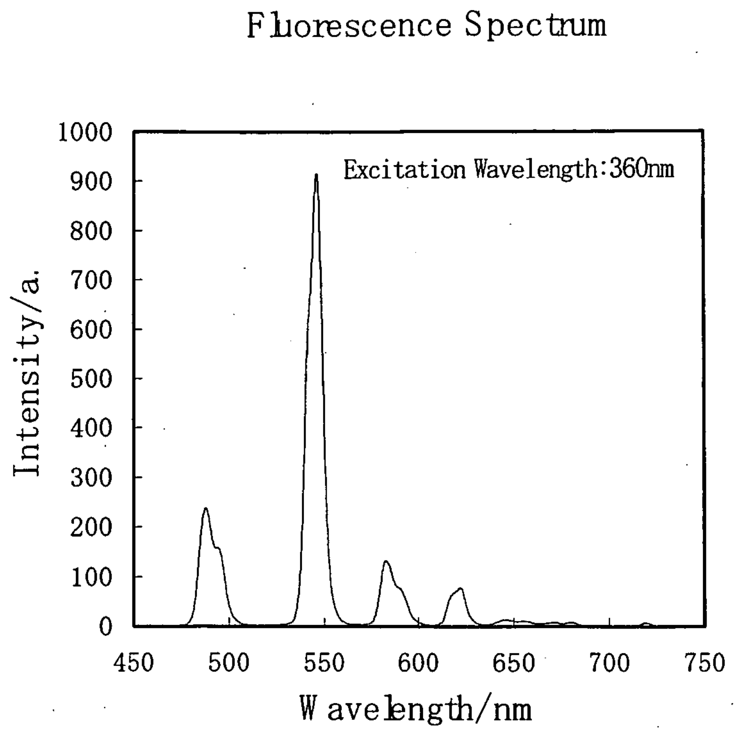
Fluorescence Spectrum
Excitation Wavelength:360nm
Intensity/a.
1000
900
800
700
600
500
400
300
200
100
0
450
500
550
600
650
700
750
Wavelength/nm

Fig. 6

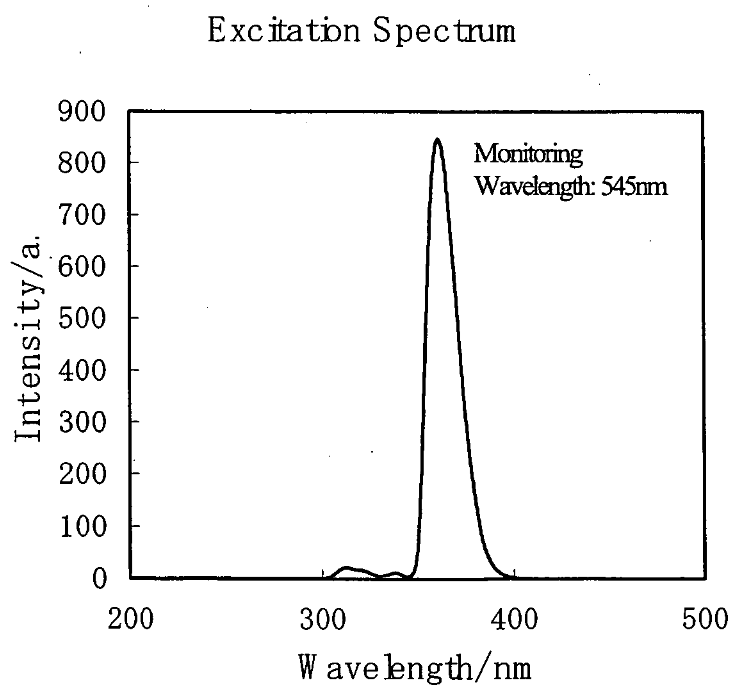
Excitation Spectrum
Monitoring
Wavelength: 545nm
Intensity/a.
900
800
700
600
500
400
300
200
100
0
200
300
400
500
Wavelength/nm

Fig. 7

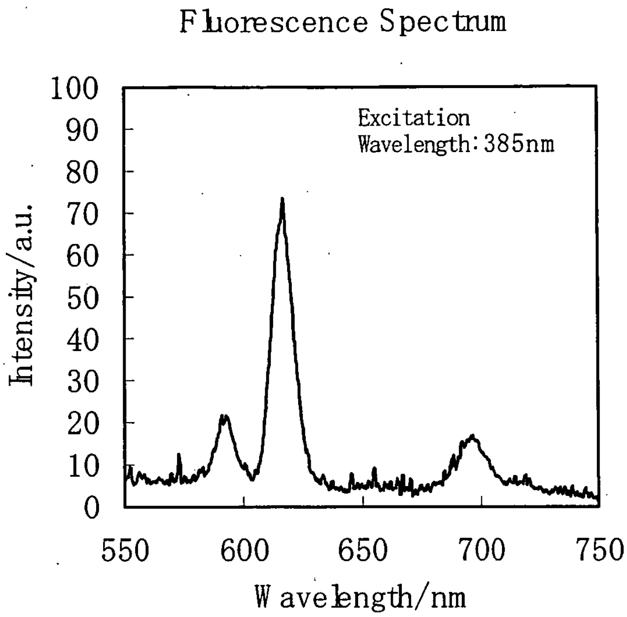
Fluorescence Spectrum
Excitation
Wavelength: 385nm
Intensity/a.u.
100
90
80
70
60
50
40
30
20
10
0
550
600
650
700
750
Wavelength/nm

Fig. 8

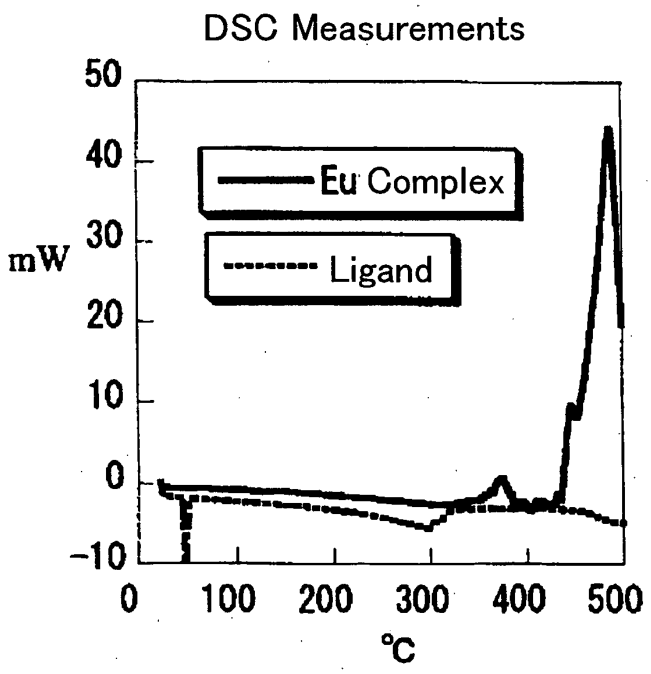
DSC Measurements
Eu Complex
Ligand
mW
50
40
30
20
10
0
-10
0
100
200
300
400
500
°C

## INTERNATIONAL SEARCH REPORT

International application No.
PCT/JP2004/014848

A. CLASSIFICATION OF SUBJECT MATTER
Int.Cl[7] C07C49/92, 69/88, C08K5/00, C08L101/00//C07F5/00, C09K11/06

According to International Patent Classification (IPC) or to both national classification and IPC

B. FIELDS SEARCHED

Minimum documentation searched (classification system followed by classification symbols)
Int.Cl[7] C07C49/92, 69/88, C08K5/00, C08L101/00, C07F5/00, C09K11/06

Documentation searched other than minimum documentation to the extent that such documents are included in the fields searched

Electronic data base consulted during the international search (name of data base and, where practicable, search terms used)
CAPLUS(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>A | GAJADHAR-PLUMMER, Alison S. et al., One-Pot Synthesis, Structure, and Unusual Luminescence of Novel One-Dimensional Lanthanide(III) Tetramethoxyborates, Inorganic Chemistry, 1999, Vol.38, No.8, pages 1745 to 1753, Tables 1, 2 | 1,3,9,13-14<br>2,4-8,10-12 |
| X<br>A | BRZYSKA, W. et al., Thermal decomposition of rare earth element 2,4-dihydroxybenzoates in air, Thermochimica Acta, 1992, No.211, pages 199 to 207, TABLES 1, 2, 3 | 1,3,9<br>2,4-8,10-14 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 17 March, 2005 (17.03.05) | 29 March, 2005 (29.03.05) |
| Name and mailing address of the ISA/<br>Japanese Patent Office<br>Facsimile No. | Authorized officer<br><br>Telephone No. |

Form PCT/ISA/210 (second sheet) (January 2004)

**INTERNATIONAL SEARCH REPORT**

International application No.
PCT/JP2004/014848

Although claims 1-2, 9 and 13-14 relate to a complex having "a photosensitizing function" and/or "a vibrational energy deactivation suppressing function", only a part of the claimed compounds is supported by the description within the meaning of PCT Article 6 and disclosed in the description within the meaning of PCT Article 5.

Consequently, this international search report regarding claims 1-2, 9 and 13-14 covers only the compound of claim 3 which is specifically disclosed in the description and the compounds mentioned as examples in paragraphs [0024] and [0025].

Form PCT/ISA/210 (extra sheet) (January 2004)